# EUROPÄISCHE PATENTANMELDUNG

(11) **EP 2 332 497 A2**
(43) Veröffentlichungstag der Anmeldung: **15.06.2011**
(21) Anmeldenummer: 10194214.2
(22) Anmeldetag: 08.12.2010
(51) Int. Cl.: A61F 2/50, A61F 2/76, A61F 2/60

(54) **Vorrichtung und Verfahren zur Befestigung einer Anschlussvorrichtung für eine Prothese und/oder einen Prothesenadapter an einem Prothesenschaft und Schutzumhüllung**

(30) Priorität: 11.12.2009 DE 102009057897
(71) Anmelder: medi GmbH & Co. KG, 95448 Bayreuth (DE)
(72) Erfinder:
(74) Vertreter: Blaumeier, Jörg

(57) **Zusammenfassung**

Vorrichtung (1) zur Befestigung einer Anschlussvorrichtung (13) für eine Prothese und/oder einen Prothesenadapter (37) an einem Prothesenschaft (11), welche:
- eine erste Aufnahme (10) für den Prothesenschaft (11),
- eine zweite, insbesondere unterhalb der ersten Aufnahme (10) angeordnete Aufnahme (12) für die Anschlussvorrichtung (13),
- Mittel zur relativen Verschiebung und/oder Verschwenkung des in der ersten Aufnahme (10) fixierten Prothesenschafts (11) und der in der zweiten Aufnahme (12) fixierten Anschlussvorrichtung (13) und
- Mittel zur festen Verbindung des fixierten Prothesenschafts (11) und der fixierten Anschlussvorrichtung (13) in einer gewünschten relativen Position und/oder Ausrichtung aufweist.

## Beschreibung

Die Erfindung betrifft eine Vorrichtung und ein Verfahren zur Befestigung einer Anschlussvorrichtung für eine Prothese und/oder einen Prothesenadapter an einem Prothesenschaft sowie eine zugehörige Schutzumhüllung.

Bei heutigen Prothesen ist es bekannt, zunächst eine Anschlussvorrichtung mit dem Prothesenschaft zu verbinden. Häufig wird dabei eine Anschlussvorrichtung für einen modularen Prothesenadapter verwendet, so dass es möglich ist, verschiedene Prothesen anzuschließen. Vorgehensweisen zur Verbindung eines Prothesenschafts mit einer solchen Anschlussvorrichtung sind im Stand der Technik vor allem für Beinprothesen bekannt.

Dabei sind im Wesentlichen zwei verschiedene Varianten zur Verbindung des Prothesenschafts mit den distalen Passteilen bekannt.

Eine Möglichkeit ist, einen Prothesenadapter direkt mit dem Prothesenschaft zu verbinden. Bei Protheseninterimsschäften aus Kunststoff ist es bekannt, Adapter aufzukleben und/oder zu verschrauben. Bei Definitivschäften, insbesondere aus laminierten Kompositmaterialien, kann der Adapter beispielsweise mit eingelegt und vergossen werden. Diese Verbindungsart hat den Nachteil, dass sie sich nur für relativ gerade Amputationsstümpfe mit leichter Beugekontraktur eignet. Bei stärkeren Beugekontrakturen ist es nicht mehr möglich, durch zweimaliges Verkippen von modularen Verbindungen einen korrekten statischen Prothesenaufbau zu gewährleisten. Dann muss der Punkt des Prothesenschlusses zum Stumpfende hin horizontal verschoben am Schaft angebracht werden.

Daher eignet sich eine Verbindung mittels eines Prothesenadapters nur für Amputierte, die einen relativ beugefreien (geraden) Prothesenaufbau benötigen. Eine Verwendung von zusätzlichen Adaptern, die die Stellung des Prothesenschaftes in horizontaler Richtung verändern, verursachen ein höheres Gewicht und einen höheren Preis sowie selbstverständlich auch einen höheren Aufwand.

Ein weiterer Nachteil dieses beschriebenen ersten bekannten Verfahrens ist es, dass sich die Höhe der Anordnung des Prothesenadapters nicht variieren lässt. Man fixiert den Prothesenadapter grundsätzlich direkt unter dem Prothesenschaft. Vor allem beim Bau von Oberschenkelprothesen ist es mitunter nötig, den Adapteranschlusspunkt nach distal verschoben am Schaft anzubringen, um das Prothesenkniegelenk, ohne den Einsatz zusätzlicher Adapter, direkt so befestigen zu können, dass der Drehpunkt des Prothesenkniegelenkes sich auf Höhe des Kniedrehpunktes der kontralateralen Seite befindet.

Aus diesem Grund wird bei einer zweiten gebräuchlichen Variante zur Erstellung von Prothesenschäften der Schaft durch Angießen von Polyurethan-Schaum (PU-Schaum) verlängert. Anschlie0end wird der erhärtete Schaum derart gekürzt, dass der Adapter in einer Position an den Schaum geklebt werden kann, deren Verkippung und Verschiebung in x, y und Z-Achse zur prothetischen Versorgung geeignet ist.

Diese Verbindung wird in der Regel auf folgende Weise hergestellt: Der Prothesenschaft wird umgedreht und auf das distale Ende wird eine konische oder zylindrische Hülle gesteckt. Danach wird ein aus zwei Komponenten zusammen zu mischender PU-Schaum flüssig in den umhüllten Bereich gegossen. Nach dem Expandieren und Aushärten des PU-Schaums wird dieser auf die gewünschte Höhe und Schaftbeugestellung heruntergeschliffen. Anschließend wird eine Anschlussvorrichtung in der gewünschten Position am Schaum befestigt. Diese Verbindung kommt normalerweise durch Verkleben zu Stande. Ist es nötig, die horizontale Position der Anschlussvorrichtung relativ weit zu ändern, kann der PU-Schaum auch leicht durchtrennt und in einer anderen Position erneut zusammengefügt werden.

Allerdings ist die Haltbarkeit dieser Verbindung bzw. Befestigungsmethode nicht sehr groß, so dass es für eine längere Anwendung der Prothese durch den Amputierten notwendig ist, diese Verbindung zu verstärken. Dies geschieht durch ein nachträgliches Aufbringen einer äußeren Verstärkungsschicht, beispielsweise Laminatschichten oder Kunststoffbinde.

Diese Verbindung bzw. Vorgehensweise hat die folgenden Nachteile. Zunächst sind die richtige Prothesenschaftstellung und die passende Höhe der Anschlussvorrichtung bei dieser Vorgehensweise sehr schwierig herzustellen, da man sich der gewünschten relativen Position und Ausrichtung von Prothesenschaft und Anschlussvorrichtung nur langsam durch Zuschleifen annähert. Denn bei der vorzunehmenden Einstellung muss der Orthopädietechniker schrittweise zuschleifen und von außen nachmessen, ob die ideale relative Position und Ausrichtung erreicht ist. Dies ist nicht nur sehr zeitaufwendig und schwierig umzusetzen, es ist im Weiteren auch schlecht dokumentierbar.

Maßgeblich für die Haltbarkeit der Verbindung zwischen dem Prothesenschaft und der Anschlussvorrichtung ist unter anderem auch das richtige Mischungsverhältnis des PU-Schaums, welches vom Orthopädietechniker abhängig ist. Die manuell hergestellten Verbindungen sind häufig von schlechterer Qualität. Zudem bildet die Klebeverbindung zwischen der Anschlussvorrichtung und dem PU-Schaum eine ungewünschte Sollbruchstelle.

Der Erfindung liegt daher die Aufgabe zugrunde, ein Verfahren und eine Vorrichtung zur Verbindung eines Prothesenschafts mit einer Anschlussvorrichtung anzugeben, bei welchen es auf einfache Art und Weise möglich ist, unterschiedliche Geometrien, insbesondere also die relative Position und Ausrichtung von Prothesenschaft und Aufnahmevorrichtung, möglichst exakt einzustellen.

Eine zur Lösung dieser Aufgabe vorgesehene Vorrichtung zur Befestigung einer Anschlussvorrichtung für eine Prothese und/oder einen Prothesenadapter an einem Prothesenschaft zeichnet sich erfindungsgemäß aus durch:
- eine erste Aufnahme für den Prothesenschaft,
- eine zweite, insbesondere unterhalb der ersten Aufnahme angeordnete Aufnahme für die Anschlussvorrichtung,
- Mittel zur relativen Verschiebung und/oder Verschwenkung des in der ersten Aufnahme fixierten Prothesenschafts und der in der zweiten Aufnahme fixierten Anschlussvorrichtung und
- Mittel zur festen Verbindung des fixierten Prothesenschafts und der fixierten Anschlussvorrichtung in einer gewünschten relativen Position und/oder Ausrichtung.

Erfindungsgemäß wird also vorgeschlagen, eine dedizierte Vorrichtung zu schaffen, in welcher der Prothesenschaft und die Anschlussvorrichtung zunächst in jeweiligen ersten und zweiten Aufnahmen fixiert werden können. Mittels Mitteln zur relativen Verschiebung und/oder Verschwenkung der entsprechend positionierten, zu verbindenden Teile kann dann hochexakt eine gewünschte, für den Patienten ideale, endgültige relative Position und/oder Ausrichtung komfortabel eingestellt werden. Nachdem dies geschehen ist, können die erfindungsgemäß vorgesehenen Mittel zur festen Verbindung genutzt werden, um eine feste Verbindung zwischen dem noch immer in der ersten Aufnahme fixierten Prothesenschaft und der in der zweiten Aufnahme fixierten Anschlussvorrichtung zu schaffen. Das bedeutet, während die Verbindung geschaffen wird, die also die Anschlussvorrichtung letztlich an dem Prothesenschaft befestigt, bleibt die gewünschte relative Position und/oder Ausrichtung in jedem Fall erhalten, so dass ein komplexes Nachjustieren oder dergleichen ebenso vollkommen entfällt. Dabei kann die Verbindung - je nach endgültiger relativer Position und/oder Ausrichtung - eine mittelbare Befestigung oder in manchen Fällen auch eine unmittelbare Befestigung bedingen, wobei vorliegend bevorzugt die Verbindung über ein Füllmittel hergestellt wird, worauf im Folgenden noch näher eingegangen wird.

Mit der erfindungsgemäßen Vorrichtung gelingt es also erstmals, eine Apparatur zu schaffen, mit der auf einfache, zeitsparende und nachvollziehbare Art und Weise unter Herstellung einer exakten endgültigen relativen Position und/oder Ausrichtung eine Verbindung zwischen einem Prothesenschaft und einer Anschlussvorrichtung für eine Prothese und/oder einen Prothesenadapter geschaffen werden kann, so dass die Bedürfnisse eines amputierten Patienten in Bezug auf die Prothesenstatik erfüllt werden können und eine Grundlage für eine verlässliche, stabile und korrekt ausgerichtete, also komfortabel nutzbare Prothese geschaffen wird.

Beispielsweise im Fall einer Oberschenkelprothese ist es besonders vorteilhaft, wenn die erste Aufnahme oberhalb der zweiten Aufnahme angeordnet ist. Dann kann beispielsweise vorgesehen sein, dass der Amputierte seinen Prothesenschaft von oben in die erste Aufnahme einbringt, wo er fixiert wird. Dabei kann beispielsweise eine vordefinierte Position und/oder Ausrichtung gewählt werden. Beispielsweise kann zunächst der Prothesenschaft vertikal ausgerichtet sein und sich exakt mittig über der horizontal eben ausgerichteten, in der zweiten Aufnahme fixierten Aufnahmevorrichtung befinden. Dann können gegebenenfalls Einstellungen vorgenommen werden, wenn eine Abweichung von dieser vordefinierten Position und/oder Ausrichtung erforderlich ist. Auch wenn im Folgenden häufig eine Beinprothese als Beispiel hergenommen wird, sei doch hervorgehoben, dass die erfindungsgemäße Vorrichtung auch für andere Prothesen grundsätzlich geeignet ist.

Wie bereits angedeutet, ist es von besonderem Vorteil, wenn die erste Aufnahme zur im Wesentlichen vertikalen Fixierung des Prothesenschafts ausgebildet ist, da dann - insbesondere im Fall einer Beinprothese - die Prothesenlängsrichtung der Vertikalen entspricht, die im Folgenden auch die Vorzugsausrichtung der Prothese, insbesondere bei Betrachtung der Statik, ist.

In weiterer Ausgestaltung der vorliegenden Erfindung kann vorgesehen sein, dass die Mittel zur festen Verbindung eine insbesondere an der Anschlussvorrichtung, insbesondere mittels eines einer Nut der Anschlussvorrichtung entsprechenden Steges, befestigbare, den Freiraum zwischen dem in der ersten Aufnahme fixierten Prothesenschaft und der in der zweiten Aufnahme fixierten Anschlussvorrichtung abdichtende, flexible Verbindungshülse umfassen. Diese Verbindungshülse dient besonders vorteilhaft mehreren Zwecken, insbesondere, wenn sie bereits vor der relativen Verschiebung und/oder Verschwenkung genutzt wird. Sie bildet zum einen eine Form für ein später zur Bildung der Verbindung zu verwendendes Füllmittel, insbesondere einen PU-Schaum, der aufgrund ihrer den Freiraum abdichtenden Wirkung im für die Verbindung vorgesehenen Freiraum verbleibt. Die Verbindungshülse ist dabei flexibel ausgebildet, so dass eine relative Verschiebung und/oder Verschwenkung der fixierten Teile auch weiterhin möglich bleibt. Zum anderen bildet die Verbindungshülse einen optischen Anhaltspunkt bezüglich des späteren Aussehens der Verbindung, so dass dem die korrekte relative Position und/oder Ausrichtung suchenden Benutzer, insbesondere einem Orthopädietechniker, auch ein optischer Anhaltspunkt gegeben wird, der ihm eine noch genauere und exaktere Einstellung der zukünftigen Prothesenstatik erlaubt. Hierbei ist es selbstverständlich von besonderem Vorteil, wenn die Verbindungshülse eine der gewünschten endgültigen Form der Verbindung entsprechende Formgebung aufweist, insbesondere eine Kegelstumpfform und/oder eine Zylinderform. Dann muss zum einen nach dem Einfüllen und Aushärten des Füllmittels, welches die Verbindung bildet, keine Nachbearbeitung mehr vorgenommen werden, zum anderen wird der optische Hinweis, den der Benutzer durch die Verbindungshülse erhält, verbessert. Dabei sind sowohl im Wesentlichen zylindrische, also schlauchartige Formen wie auch die Form eines Kegelstumpfs, also leicht konisch zulaufend, vorteilhaft denkbar.

Wie bereits erwähnt, ist es möglich, die Verbindungshülse an der Anschlussvorrichtung zu befestigen, wobei darauf hingewiesen sei, dass selbstverständlich auch eine anderweitige Befestigung denkbar ist, eine Befestigung an der Anschlussvorrichtung sich jedoch insbesondere anbietet, wenn die zweite Aufnahme unterhalb der ersten Aufnahme angeordnet ist, da dann dort die hauptsächliche abdichtende Wirkung benötigt wird. Dazu kann beispielsweise die Anschlussvorrichtung eine insbesondere am Außenumfang der Anschlussvorrichtung umlaufende Nut umfassen, wobei die Anschlussvorrichtung zweckmäßigerweise im Wesentlichen rund geformt ist. Dieser Nut entsprechend ist am Rand der Verbindungshülse ein Steg angeformt, der beim Aufstülpen und/oder Aufrollen der Verbindungshülse in die Nut insbesondere passgenau eingreift. Die Befestigung kann beispielsweise durch ein Verkleben verbessert werden. Die Höhe der Verbindungshülse ist dabei im Allgemeinen so gewählt, dass verschiedene Abstände zwischen der Anschlussvorrichtung und dem Prothesenadapter, wie sie bei verschiedenen Patienten vorkommen können, eingestellt werden können und dennoch noch der Freiraum vollständig abgedeckt bleibt.

Wie bereits erwähnt, können die Mittel zur festen Verbindung ferner ausgebildet sein, den durch die Verbindungshülse abgedichteten Freiraum mit einem Füllmittel, insbesondere einem PU-Schaum, auszufüllen, der dann aushärtet. Hierzu können die Mittel zur festen Verbindung eine Spritzvorrichtung und einen Schlauch und/oder ein Rohr zur Zuführung des Füllmittels in den Freiraum umfassen. Während es grundsätzlich selbstverständlich vorgesehen sein kann, den Schlauch bzw. das Rohr zur Zuführung des Füllmittels und/oder eine entsprechende Entlüftungsvorrichtung einfach von oben zur Herstellung einer Verbindung mit dem Freiraum einzuschieben, beispielsweise zwischen Prothesenschaft und Verbindungshülse, ist es jedoch mit besonderem Vorteil möglich, die Verbindungshülse, die dann insbesondere als Teil der erfindungsgemäßen Befestigungsvorrichtung anzusehen ist, bereits für diese Funktion auszugestalten.

So kann die Verbindungshülse eine Einlassöffnung zum Einleiten eines den Prothesenschaft mit der Anschlussvorrichtung verbindenden Füllmittels, insbesondere eines PU-Schaums, aufweisen. An der Einlassöffnung kann zudem ein Anschluss für einen das Füllmittel zuführenden Schlauch und/oder ein das Füllmittel zuführendes Rohr, welches, wie oben beschrieben, Teil der Mittel zur festen Verbindung sein kann, vorgesehen sein. Die Einlassöffnung kann dabei, insbesondere im eingesetzten Zustand der Verbindungshülse, im unteren Bereich angeordnet sein. Mit besonderem Vorteil kann die Einlassöffnung eine insbesondere auf einer elastischen Kraft basierende Schließvorrichtung umfassen, so dass die Einlassöffnung beispielsweise von außen mit einem Stutzen geöffnet werden kann und sich bei Entfernung des Stutzens automatisch wieder verschließt. Ist ein zweikomponentiges Füllmittel, beispielsweise ein zweikomponentiger PU-Schaum, als Füllmittel vorgesehen, so kann zweckmäßigerweise im Schlauch und/oder Rohr eine Mischerkartusche zum Vermischen des zweikomponentigen Füllmittels vorgesehen sein. Selbstverständlich ist es jedoch auch denkbar, dass eine Mischung des Füllmittels in der Spritzvorrichtung durchgeführt wird. Schließlich ist es aber auch denkbar, das zweikomponentige Füllmittel in einem separaten Behälter zu mischen und anschließend in die Spritzvorrichtung einzufüllen. Ein Mischen in der Spritzvorrichtung kann durch manuelles Schütteln erreicht werden, genauso gut ist es jedoch denkbar, eine automatische Mischvorrichtung als Teil der Spritzvorrichtung vorzusehen. Grundsätzlich kann im Rahmen der vorliegenden Erfindung selbstverständlich auch ein einkomponentiges Füllmittel benutzt werden.

Mit besonderem Vorteil kann die Verbindungshülse ferner wenigstens eine insbesondere höher als die Einlassöffnung angeordnete Luftauslassöffnung zum Auslassen von Luft bei Einleiten des Füllmittels aufweisen. Dazu ist auch ein Luftauslass bereits in die Verbindungshülse integriert, so dass ein zusätzlicher, beispielsweise, wie beschrieben, am oberen Ende einzuführender Schlauch nicht mehr erforderlich ist. Vorzugsweise können mehrere, insbesondere gleichmäßig verteilte, eine einen Austritt von Luft erlaubende und einen Austritt von Füllmittel verhindernde Größe aufweisende Luftauslassöffnungen vorgesehen sein. Beispielsweise können zirkulär umlaufend auf verschiedenen Höhen der Verbindungshülse kleine Löcher angeordnet sein, welche das Ausströmen der Luft erlauben, aber aufgrund ihrer geringen Größe das Füllmittel (Verbindungsmasse) nicht hinausströmen lassen. Erreicht das Füllmittel die Löcher bzw. Luftauslassöffnungen, so werden diese durch das Füllmittel verschlossen. In einer alternativen Ausgestaltung kann jedoch auch vorgesehen sein, dass die Luftauslassöffnungen an wenigstens einen innerhalb der Verbindungshülse verlaufenden Kanal angeschlossen sind, welcher insbesondere durch wenigstens eine weitere Öffnung mit der Umgebung verbunden ist. Beispielsweise können auch diese Kanäle im Einbauzustand im Wesentlichen vertikal verlaufen. Für die Luftauslassöffnungen gilt auch hier, dass sie vorteilhafterweise von einer den Austritt von Luft erlaubenden und den Austritt von Füllmittel verhindernden Größe sind, folglich dann auch von dem Füllmittel verschlossen werden können.

Zweckmäßigerweise besteht die Verbindungshülse aus einem im Wesentlichen keine Haftverbindung mit dem Füllmittel eingehenden Material. Dann kann sie nach dem Einfüllen und Erhärten des Füllmittels wieder abgenommen werden und steht folglich für weitere Verwendungen als Teil der erfindungsgemäßen Vorrichtung weiter zur Verfügung. Dies ist besonders wünschenswert, wenn Einlass- bzw. Auslassöffnungen in die Konstruktion der Verbindungshülse integriert sind. Es sei jedoch bereits jetzt darauf hingewiesen, dass in einer alternativen Ausgestaltung die Verbindungshülse als als Teil der Verbindung verbleibende Umhüllung, insbesondere als Schutz- und/oder Verstärkungsumhüllung, ausgebildet ist und somit nach dem Einfüllen und Erhärten des Füllmittels als ein Teil der Verbindung zwischen Prothesenschaft und Anschlussvorrichtung verbleibt. Jedoch kann es zweckmäßiger sein, die Verbindungshülse abnehmbar zu gestalten, denn dann kann beispielsweise bei einer dennoch vorkommenden ungünstigen relativen Position und Ausrichtung die Verbindung abgesägt und nochmals vorgenommen werden.

In weiterer vorteilhafter Ausgestaltung der vorliegenden Erfindung kann vorgesehen sein, dass die Mittel zur relativen Verschwenkung und/oder Verschiebung eine Höheneinstelleinrichtung für die erste Aufnahme und/oder wenigstens eine Schwenkvorrichtung für die erste Aufnahme umfassen. Dadurch lässt sich die erste Aufnahme und mithin der Prothesenschaft von der Ebene der in der zweiten Aufnahme fixierten Anschlussvorrichtung beabstanden bzw. kann an diese angenähert werden, beispielsweise, um die korrekte Lage eines Kniegelenks auf der Höhe des gesunden Kniegelenks einstellen zu können. Zudem kann eine Schwenkvorrichtung für die erste Aufnahme vorgesehen sein, die insbesondere eine Verschwenkung um beliebige horizontale Achsen erlaubt, wenn der Prothesenschaft im Wesentlichen vertikal ausgerichtet fixiert ist. Auf diese Weise können beispielsweise Beugekontrakturen oder dergleichen ausgeglichen werden. Ergänzt werden kann eine solche Ausgestaltung ideal dadurch, dass die Mittel zur relativen Verschwenkung und/oder Verschiebung insbesondere zusätzlich eine Einstelleinrichtung zur horizontalen Verschiebung der zweiten Aufnahme gegen die erste Aufnahme umfassen, so dass dann, beispielsweise nach einer Verschwenkung des vertikal fixierten Prothesenschafts in der ersten Aufnahme um eine horizontale Achse, auch eine Anpassung der Lage der Anschlussvorrichtung senkrecht zur Ausrichtung des Prothesenschafts vorteilhaft erfolgen kann.

Dabei kann die Höhenverstelleinrichtung beispielsweise ein Stativ, gebaut aus hohlen Profilen, mit einer darin geführten, höhenverstellbaren Stange aufweisen, die die erste Aufnahme trägt. Über eine Schraube, die an der Stange angreift, kann eine Fixierung der ersten Aufnahme in einer bestimmten Höhe erreicht werden. Das Stativ kann dabei beispielsweise neben einem Hohlrohr, in dem die höhenverstellbare Stange geführt ist, eine Grundplatte aufweisen, auf der dann - gegebenenfalls über die Einstelleinrichtung horizontal verschiebbar geführt - die zweite Aufnahme angeordnet ist. Das Stativ und die Grundplatte verbinden somit die erste Aufnahme und die zweite Aufnahme, die folglich nur definiert über die Mittel zur relativen Verschwenkung und/oder Verschiebung in ihrer relativen Position und/oder Ausrichtung verändert werden können. In einer Ausführungsform kann zudem vorgesehen sein, dass die erste Aufnahme durch eine lösbare Klemmvorrichtung als Teil der Schwenkvorrichtung drehbar an der Stange geführt ist. Auf diese Weise kann beispielsweise eine Verschwenkung um eine vertikale Achse realisiert werden, wenn der Prothesenschaft vertikal fixiert ist. Eine derartige Verschwenkung wird jedoch nicht in jedem Fall notwendig sein, nachdem sich eine derartige Bewegung auch durch die Einstelleinrichtung zur horizontalen Verschiebung der zweiten Aufnahme gegen die erste Aufnahme abbilden lässt.

Vorzugsweise kann jedoch die Schwenkverbindung eine Gelenklagerung der ersten Aufnahme umfassen, insbesondere eine zwischen der ersten Aufnahme und der Klemmvorrichtung angeordnete Gelenkverbindung zum Verschwenken um wenigstens eine vertikale Achse. Eine derartige Gelenkverbindung kann beispielsweise als ein Kugelgelenk ausgebildet sein.

Sonstige Mittel zur Realisierung der Einstelleinrichtung, der Schwenkvorrichtung und der Höhenverstelleinrichtung sind im Stand der Technik weitgehend bekannt und können selbstverständlich auch bei der erfindungsgemäßen Befestigungsvorrichtung genutzt werden.

Es sei an dieser Stelle noch angemerkt, dass auch als erste und zweite Aufnahme im Rahmen der vorliegenden Erfindung beliebige Einrichtungen benutzt werden können, die ihren Zweck erfüllen, den Prothesenschaft bzw. die Anschlussvorrichtung zu fixieren. Beispielsweise kann bezüglich des Prothesenschafts als Aufnahme ein den Prothesenschaft umgreifender Klemmring vorgesehen werden. Die zweite Aufnahme kann beispielsweise derart ausgebildet sein, dass sie die an der Anschlussvorrichtung ohnehin vorgesehenen Befestigungsmittel für die Prothese und/oder den Prothesenadapter nutzt, um eine Fixierung zu erreichen.

Mit besonderem Vorteil kann vorgesehen sein, dass wenigstens eine Messvorrichtung zur Anzeige einer eingestellten relativen Positionierung und/oder Ausrichtung vorgesehen ist. Unter Nutzung derartiger Messvorrichtungen ist es möglich, genau zu dokumentieren, welche relative Position und/oder Ausrichtung von Prothesenschaft und Anschlussvorrichtung am Ende gewählt wurde, um die befestigende Verbindung zu schaffen. Es ist folglich eine weitaus verbesserte Möglichkeit zur Dokumentation der Anpassung der Prothesenteile gegeben, die vorteilhaft bei weiteren Anpassungen und/oder neuen Prothesen beim Patienten genutzt werden kann. Insbesondere kann vorgesehen sein, dass als Messvorrichtung wenigstens eine Skala, insbesondere eine auf einer in einem Hohlstativ geführten Stange angeordnete und/oder eine an einer Führung eines Klemmrings einer an einer Stange angreifenden Klemmvorrichtung und/oder wenigstens eine einer Einstellvorrichtung zur horizontalen Verschiebung der zweiten Aufnahme zugeordnete Skala, und/oder wenigstens ein Winkelmesser, insbesondere wenigstens ein, insbesondere zwei, an der ersten Aufnahme angeordnete Winkelmesser zur Messung eines Schwenkwinkels um eine horizontale Achse, vorgesehen sein. Dabei sei darauf hingewiesen, dass viele im Stand der Technik bekannte Mittel zur relativen Verschiebung und/oder Verschwenkung grundsätzlich bereits eine eingebaute Messvorrichtung umfassen, die insbesondere exakt angibt, welche Einstellung gerade vorliegt. Beispielsweise können aufgedruckte Skalen, wie beschrieben, vorgesehen sein, oder aber auch integrierte Winkelmesser bei Verschwenkeinrichtungen. Ist eine Schwenkvorrichtung als Gelenkverbindung vorgesehen, so können mit besonderem Vorteil Winkelmesser eingesetzt werden. Diese können beispielsweise an der ersten Aufnahme selbst vorgesehen sein. Ein Winkelmesser kann dabei ein der Gravitationskraft unterworfenes Lot und/oder einen auf die Längsrichtung des Prothesenschafts einstellbaren Zeiger umfassen. Denkbar ist aber auch, das Lot durch einen manuell in Vertikalrichtung einzustellenden Zeiger zu ersetzen. So kann dann direkt abgelesen werden, inwieweit gegenüber der Vertikalen der Prothesenschaft verschwenkt ist. Selbstverständlich sind jedoch auch andere Ausgestaltungen denkbar.

Neben der Vorrichtung betrifft die Erfindung auch ein Verfahren zur Befestigung einer Anschlussvorrichtung für eine Prothese und/oder einen Prothesenadapter an einem Prothesenschaft, wozu mit besonderem Vorteil die erfindungsgemäße Vorrichtung verwendet werden kann. Dieses Verfahren zeichnet sich dadurch aus, dass
- die Anschlussvorrichtung und der Prothesenschaft in einer definierten relativen Position und/oder Ausrichtung zueinander jeweils in einer zweiten und einer ersten Aufnahme fixiert werden,
- durch relative Verschiebung und/oder Verschwenkung der Aufnahmen zueinander eine endgültige relative Position und/oder Ausrichtung eingestellt wird, und
- die fixierte Anschlussvorrichtung und der fixierte Prothesenschaft unter Bildung einer festen Verbindung aneinander in der endgültigen relativen Position und/oder Ausrichtung befestigt werden.

Mit dem erfindungsgemäßen Verfahren, welches eine einzige Vorrichtung nutzt, um die passende Einstellung der relativen Positionierung und/oder Ausrichtung zu erlauben und die Verbindung zu realisieren, können die mit der erfindungsgemäßen Vorrichtung erzielbaren Vorteile erreicht werden. Dabei sei hervorgehoben, dass sich die bezüglich der erfindungsgemäßen Vorrichtung getätigten Ausführungen selbstverständlich analog auf das erfindungsgemäße Verfahren übertragen lassen.

Zunächst werden also sowohl der Prothesenschaft als auch die Anschlussvorrichtung in einer ersten Aufnahme bzw. einer zweiten Aufnahme einer Befestigungsvorrichtung eingespannt. Aufgrund geeignet ausgebildeter Mittel zur relativen Verschiebung und/oder Verschwenkung der in den Aufnahmen fixierten Teile kann nun die gewünschte relative Position und/oder Ausrichtung eingestellt werden, insbesondere also die gewünschte Höhe und Stumpfstellung (Ab- und Adduktion, Flexion und Extension). Ist die endgültige, also insbesondere die für den Patienten ideale, relative Position und/oder Ausrichtung erreicht, kann insbesondere durch ebenso an der Befestigungsvorrichtung vorgesehene Mittel zur festen Verbindung des fixierten Prothesenschafts und der fixierten Anschlussvorrichtung eine feste Verbindung geschaffen werden, die die Anschlussvorrichtung an dem Prothesenschaft in der gewünschten endgültigen relativen Position und/oder Ausrichtung befestigt. So ist ein einfache und exakte Befestigung gegeben, die die Prothesenstatik und die individuellen Eigenschaften des Patienten berücksichtigt.

In Weiterbildung des Verfahrens kann vorgesehen sein, dass die erste, oberhalb der zweiten Aufnahme liegende erste Aufnahme, insbesondere bei in vertikaler Richtung fixiertem Prothesenschaft, verschwenkt und/oder höhenverstellt wird und/oder die zweite Aufnahme in einer horizontalen Ebene verschoben wird. Der Prothesenschaft kann also so fixiert werden, dass seine Längsrichtung im Wesentlichen der Vertikalen entspricht und die zweite Aufnahme mit der Anschlussvorrichtung unterhalb positioniert ist. Über die Höhenverstellung kann dann eine Anpassung der Höhe eines folgenden Gelenks, beispielsweise eines Kniegelenks, angepasst werden, über die Verschwenkung, welche vorteilhafterweise um eine beliebige horizontale Achse möglich ist, können Fehlstellungen, beispielsweise Beugekontrakturen, berücksichtigt werden. Schließlich ist es noch möglich, die horizontale relative Lage von Prothesenschaft und Anschlussvorrichtung an die Gegebenheiten des Patienten anzupassen, indem die zweite Aufnahme in einer horizontalen Ebene, beispielsweise auf einer Grundplatte, verschiebbar ist.

Vorzugsweise können mittels wenigstens einer Messvorrichtung die endgültige relative Position und/oder Ausrichtung beschreibende Geometriedaten gemessen und/oder abgelesen werden. So ist nicht nur eine möglichst exakte Einstellung möglich, diese kann auch, beispielsweise für eine spätere Verwendung, exakt dokumentiert werden. Beispiele für derartige Messvorrichtungen können auf gegeneinander verschiebbaren Teilen angebrachte Skalen sein, beispielsweise eine auf der Grundplatte vorgesehene Skala, und/oder auch Winkelmesser, wie bezüglich der erfindungsgemäßen Vorrichtung bereits beschrieben.

Mit besonderem Vorteil kann ein zwischen der fixierten Anschlussvorrichtung und dem fixierten Prothesenschaft liegender Freiraum mit einem Füllmittel, insbesondere einem PU-Schaum, aufgefüllt werden, welches im ausgehärteten Zustand wenigstens einen Teil der Verbindung bildet. Dabei kann, wie bereits bezüglich der Vorrichtung beschrieben, als Form für das Füllmittel eine insbesondere zylindrische und/oder kegelstumpfförmige, elastische Verbindungshülse zur Abdichtung des Freiraums verwendet und insbesondere an der Anschlussvorrichtung, insbesondere mittels eines einer an der Anschlussvorrichtung ausgebildeten Nut entsprechenden Steges, befestigt werden. Wie oben beschrieben kann die Verbindungshülse dabei selbstverständlich auch eine Einlassöffnung und eine Luftauslassöffnung, insbesondere mehrere Luftauslassöffnungen, umfassen. Denkbar ist jedoch auch, beispielsweise einen Schlauch oder dergleichen von oben, beispielsweise zwischen Verbindungshülse und Prothesenschaft, einzuführen. Dabei wird die Verbindungshülse mit besonderem Vorteil bereits vor der Verschiebung und/oder Verschwenkung über die Anschlussvorrichtung und den Prothesenschaft gestülpt oder gerollt, so dass dem das Verfahren Durchführenden bereits ein optischer Anhalt gegeben wird, welche Form letztendlich resultieren wird. Durch die elastische Ausgestaltung der Verbindungshülse ist eine Änderung der relativen Position und/oder Ausrichtung auch weiterhin möglich. Die Verbindungshülse kann, wie beschrieben, zylinderförmig oder konisch leicht zulaufend sein, wobei ihr unterer Durchmesser idealerweise dem Durchmesser der Anschlussvorrichtung, die ebenso rund ausgebildet sein kann, entspricht.

Als Verbindungsmasse kann zweckmäßigerweise ein zweikomponentiges Füllmittel, insbesondere ein zweikomponentiger PU-Schaum, verwendet werden, welches insbesondere über eine Zweikomponentenkartusche in einem Mischerrohr und/oder in einem externen Behälter und/oder in einer Spritzvorrichtung gemischt wird. Dabei kann das Mischen in der Spritzvorrichtung beispielsweise durch deren Schütteln erreicht werden, es ist jedoch auch möglich, dass die Spritzvorrichtung eine geeignete Mischvorrichtung umfasst. Das so angemischte zweikomponentige Füllmittel kann dann durch die Einlassöffnung der Verbindungshülse bzw. auf anderem Wege eingefüllt werden, wobei die Luft durch die Luftauslassöffnungen oder auf anderem Wege entweicht. Grundsätzlich ist es jedoch auch denkbar, ein einkomponentiges Füllmittel, beispielsweise einen einkomponentigen PU-Schaum zu verwenden.

Nach dem Einfüllen härtet der Schaum innerhalb des durch die Verbindungshülse abgedichteten Freiraums aus und bildet somit eine stabile, feste Verbindung, welche den Prothesenschaft und die Anschlussvorrichtung verbindet und somit die Anschlussvorrichtung an dem Prothesenschaft befestigt.

Mit besonderem Vorteil kann vorgesehen sein, dass dem Füllmittel ein Lösungsmittel zur Verbesserung der Haftung des Füllmittels an der Anschlussvorrichtung und/oder dem Prothesenschaft zugesetzt wird. Dabei wird das Prothesenschaftmaterial und das Material der Anschlussvorrichtung im Kontaktbereich leicht angelöst, so dass sich die Haftfläche vergrößert und folglich die Haftung verbessert wird.

In einer alternativen Ausgestaltung kann vorgesehen sein, dass die zur Verbindung mit dem Füllmittel vorgesehenen Oberflächen des Prothesenschafts und/oder der Anschlussvorrichtung zur Verbesserung der Haftung angeraut werden. Auch dabei wird die Haftfläche folglich vergrößert und es wird die Haftung verbessert, was die Stabilität und Haltbarkeit der Anordnung verbessert.

Ist die Verbindungshülse vorteilhaft so ausgestaltet, dass sich im Wesentlichen keine oder nur eine sehr geringe Haftung an dem Füllmittel ergibt, so kann die Verbindungshülse nach dem Aushärten des Füllmittels abgenommen werden. Die Verbindungshülse kann dann bei weiteren Befestigungsvorgängen wieder eingesetzt werden.

Alternativ kann jedoch auch vorgesehen sein, dass die Verbindungshülse als eine Schutz- und/oder Verstärkungsumhüllung verbleibt und mithin einen Teil der Verbindung bildet. Dann ist kein Anbringen einer anderen Schutzumhüllung oder das Aufbringen einer Schicht, beispielsweise durch Laminieren oder dergleichen, notwendig.

In weiterer vorteilhafter Ausgestaltung des erfindungsgemäßen Verfahrens kann vorgesehen sein, dass zum Schutz des Füllmittels dieses mit einer Schutzumhüllung versehen wird, insbesondere ein bevorzugt harzgetränkter Schlauch oder Konus aus einem flexiblen Gewebe wenigstens auf das erhärtete Füllmittel, insbesondere auch auf die Aufnahme und/oder wenigstens einen Teil des Prothesenschafts, aufgezogen und/oder aufgerollt wird. Eine solche Schutzumhüllung, die beispielsweise nach der Überprüfung der Prothesenanstellung beim Gehen und Stehen des Amputierten aufgebracht werden kann, dient insbesondere dazu, das Füllmittel, insbesondere den PU-Schaum, vor Licht, Beschädigung und äußeren Witterungsumständen zu schützen. Dazu ist insbesondere ein harzgetränkter Schlauch oder Konus aus einem flexiblen Gewebe vorgesehen. Das Gewebe kann dabei aus transparenten Fäden und aus zugfesten Bestandteilen bestehen. Insbesondere kann die Schutzumhüllung in einem aufgerollten Zustand gelagert werden. Die aufgerollte Schutzumhüllung wird unterhalb der Aufnahme angesetzt und nach oben abgerollt, wobei das gehärtete Füllmittel, vorteilhaft aber zusätzlich die Anschlussvorrichtung und wenigstens ein Teil des Prothesenschafts, umschlossen wird. Auf diese Weise dient die Umhüllung auch der Verstärkung der Verbindung selbst. Umfasst die Anschlussvorrichtung eine Nut, die beispielsweise bereits der Befestigung der Verbindungshülse gedient hat, kann vorgesehen sein, dass die Umhüllung in der Nut der Anschlussvorrichtung mit einem insbesondere harzgetränkten Faden oder einem anderen zugfesten Faden abgebunden wird. Dieses Vorgehen dient, wie bereits erwähnt, neben dem Schutz des Füllmittels auch zur Verbesserung der Haftung zwischen Prothesenschaft und Anschlussvorrichtung sowie zur Erhöhung der Festigkeit der Verbindung.

Weiter kann vorgesehen sein, dass ein insbesondere modularer Prothesenadapter an die Anschlussvorrichtung montiert wird. Dies kann im Übrigen auch bereits vor dem Anbringen einer Umhüllung, insbesondere nach dem Aushärten des Füllmittels, geschehen. Hierzu kann die Anschlussvorrichtung beispielsweise wenigstens einen, insbesondere vier Gewindelöcher umfassen, die als Aufnahme für beispielsweise Schrauben dienen. Sind derartige Gewindelöcher durchgängig, müssen sie selbstverständlich beim Einfüllen des Füllmittels in den Freiraum verschlossen sein, beispielsweise durch einen Stopfen oder dergleichen.

Eine zur Verwendung in der erfindungsgemäßen Vorrichtung oder dem erfindungsgemäßen Verfahren vorgesehene Anschlussvorrichtung umfasst Anschlussmittel bzw. Befestigungsmittel zum Anschluss eines Prothesenadapters und/oder einer Prothese. Sie ist vorteilhaft im Wesentlichen rund ausgebildet und kann auf ihrer äußeren Oberfläche eine umlaufende Nut umfassen, die beispielsweise zur Befestigung der Verbindungshülse oder zum späteren Abbinden einer Umhüllung genutzt werden kann.

Um eine bessere Verbindung durch das Füllmittel zu erreichen, kann ferner vorgesehen sein, dass die Anschlussvorrichtung auf der im Einbauzustand dem Prothesenschaft zugewandten Seite wenigstens einen aufragenden symmetrischen und/oder asymmetrischen Steg aufweist. Dies vergrößert die Haftfläche. Vorzugsweise weist wenigstens ein Steg einen Hinterschnitt auf, so dass nicht nur eine Vergrößerung der Haftfläche auftritt, sondern auch eine Formgebung, die ein einfaches "Herausfallen" der Anschlussvorrichtung aus dem ausgehärteten Füllmittel vorteilhaft verhindert.

Wie bereits erwähnt, kann eine zum Kontakt mit einem Füllmittel vorgesehene Oberfläche der Anschlussvorrichtung aufgeraut sein, das bedeutet, dass sich hierdurch ebenso zusätzlich die Haftfläche vergrößert.

Wie ebenso bereits erwähnt, können die Anschlussmittel bzw. Befestigungsmittel der Anschlussvorrichtung wenigstens ein Gewindeloch, insbesondere vier Gewindelöcher, umfassen. Hierdurch kann beispielsweise mit Hilfe von Schrauben oder dergleichen die Prothese bzw. der Prothesenadapter an der Anschlussvorrichtung befestigt bzw. angeschlossen werden.

Schließlich betrifft die Erfindung noch eine Schutzumhüllung für eine ein ausgehärtetes Füllmaterial umfassende Verbindung eines Prothesenschafts mit einer Anschlussvorrichtung für eine Prothese und/oder einen Prothesenadapter, wobei die Verbindung insbesondere mit der erfindungsgemäßen Vorrichtung und/oder nach dem erfindungsgemäßen Verfahren hergestellt ist. Die erfindungsgemäße Schutzumhüllung zeichnet sich dadurch aus, dass die aus einem elastischen Gewebe bestehende, insbesondere schlauch- oder konusförmige Schutzumhüllung auf die Verbindung aufziehbar, insbesondere aufrollbar, ist. Eine derartige Schutzumhüllung, die folglich durch einen Gewebeschlauch oder Gewebekonus gebildet wird, kann vorteilhafterweise vorgefertigt werden und insbesondere aufgerollt gelagert vorliegen. Sie ermöglicht es auf besonders einfache Weise, einen Schutz des Füllmittels vor äußeren Einflüssen, insbesondere auch vor Licht, zu ermöglichen, indem die bereits in einem Stück zusammenhängende Schutzumhüllung einfach auf die Verbindung aufziehbar, insbesondere aufrollbar, ist. Gegenüber dem bekannten Stand der Technik, wonach beispielsweise eine Laminatschicht aufgebracht wird oder eine Kunststoffbinde um die Verbindung gewickelt wird, ist dies einfacher handzuhaben.

Bevor die Schutzumhüllung aufgebracht wird, ist oder wird sie vorzugsweise harzgetränkt, um ihre Verbindungs- bzw. Schutzeigenschaften zu verbessern. Vorteilhaft kann auch vorgesehen sein, dass sie auch zur Überdeckung wenigstens eines Teils der Anschlussvorrichtung und/oder wenigstens eines Teils des Prothesenschafts dimensioniert ist. Auf diese Weise hat sie nicht nur eine das ausgehärtete Füllmittel schützende Wirkung, sondern verstärkt auch die Verbindung zwischen dem Prothesenschaft und der Anschlussvorrichtung.

Das Gewebe kann vorzugsweise aus transparenten Fäden bestehen, um eine verbesserte Optik zu erreichen.

Es sei an dieser Stelle noch angemerkt, dass die erwähnte Anschlussvorrichtung beispielsweise auch aus einem Schaum bestehen kann. Die erfindungsgemäße Befestigungsvorrichtung, insbesondere die Aufnahmen, können vorteilhaft aus Metall gefertigt werden, es sind jedoch auch Kunststoffteile denkbar.

Weitere Vorteile und Einzelheiten der vorliegenden Erfindung ergeben sich aus den im Folgenden beschriebenen Ausführungsbeispielen sowie anhand der Zeichnung. Dabei zeigen:
- Fig. 1: eine erfindungsgemäße Befestigungsvorrichtung mit fixiertem Prothesenschaft und fixierter Anschlussvorrichtung,
- Fig. 2: eine Detailskizze zur Verwendung eines Füllmittels,
- Fig. 3: die hergestellte Verbindung mit erhärtetem Füllmittel und bereits angebrachtem Prothesenadapter,
- Fig. 4: eine erfindungsgemäße Schutzumhüllung, und
- Fig. 5: eine perspektivische Ansicht einer im Rahmen der vorliegenden Erfindung verwendbaren Anschlussvorrichtung.

Im Folgenden werden ein Ausführungsbeispiel der erfindungsgemäßen Vorrichtung und des erfindungsgemäßen Verfahrens zur Befestigung einer Anschlussvorrichtung für eine Prothese und/oder einen Prothesenadapter an einem Prothesenschaft zur Verdeutlichung der Zusammenhänge und des Gesamtvorgangs im Wesentlichen gleichzeitig beschrieben.

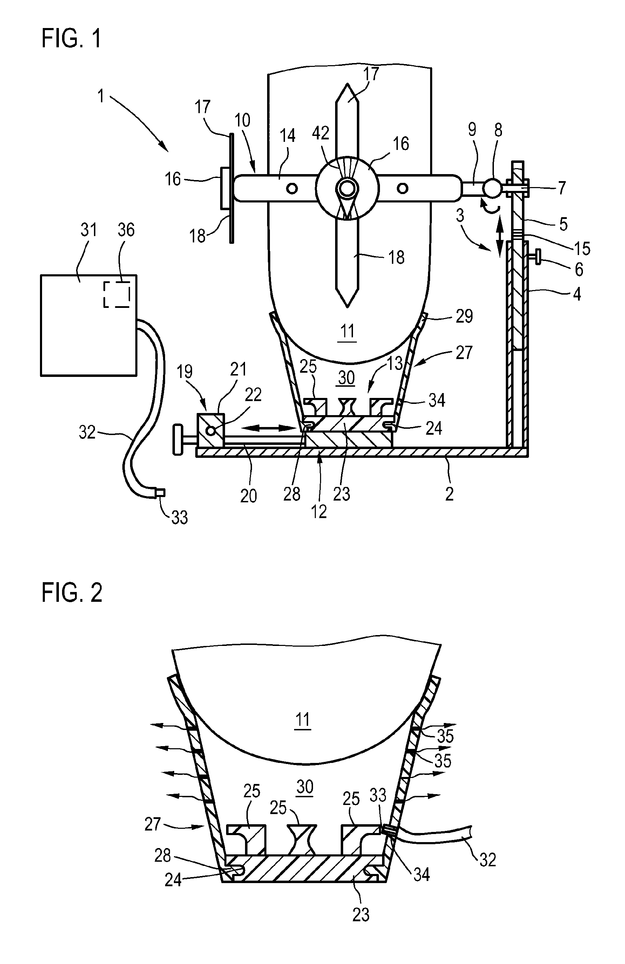

Fig. 1 zeigt - teilweise als Querschnitt - ein Ausführungsbeispiel einer erfindungsgemäßen Befestigungsvorrichtung 1, in welcher bereits ein Prothesenschaft 11 und eine Anschlussvorrichtung 13 fixiert sind.

Die Vorrichtung 1 umfasst eine metallene Grundplatte 2, auf der ein Stativ 3, umfassend ein Hohlrohr 4 und eine darin geführte Stange 5 angeordnet sind. Eine Feststellschraube 6, die an der Stange 5 angreift, ermöglicht es, eine Bewegung der Stange 5 zu verhindern. Das Stativ 3 bildet also eine Höheneinstelleinrichtung. An der Stange 5 fest befestigt ist ein erster Träger 7, der über eine Gelenkverbindung 8, beispielsweise ein Kugelgelenk, mit einem zweiten Träger 9 befestigt ist, der Teil einer ersten Aufnahme 10 für den Prothesenschaft 11 ist. Mittels der als Stativ 3 ausgebildeten Höheneinstelleinrichtung und der eine Schwenkvorrichtung bildenden Gelenkverbindung 8 ist es möglich, die Höhe der ersten Aufnahme 10 und somit eines in ihr fixierten Prothesenschafts 11 über der Grundplatte 2 zu verändern und die erste Aufnahme 10 um eine beliebige horizontale Achse zu verschwenken, so dass auch ein in ihr fixierter Prothesenschaft 11 verkippt wird. Die Gelenkverbindung 8 und das Stativ 3 sind mithin Teil von Mitteln zur relativen Verschiebung und Verschwenkung der ersten Aufnahme gegenüber einer später noch näher diskutierten, auf der Grundplatte 2 geführten zweiten Aufnahme 12 für die Anschlussvorrichtung 13.

Die erste Aufnahme 10 umfasst weiter einen hier metallenen Ring 14, der den Prothesenschaft 11 klemmend umfasst.

Auf der Stange 5 ist im Übrigen eine Skala 15 vorgesehen, mit deren Hilfe es möglich ist, die aktuelle Einstellung abzulesen, um sie dokumentieren zu können. Um die Verschwenkung über die Gelenkverbindung 8 erfassen zu können, sind zwei um 90° versetzt angeordnete Winkelmesser 16 am Metallring 14 angebracht. Diese umfassen jeweils einen Zeiger 17, der entlang der Längsachse des Prothesenschafts eingestellt ist oder wird, sowie ein Lot 18, das die Richtung der Gravitationskraft, mithin die Vertikale, anzeigt. An einer aufgebrachten Anzeige 42 lässt sich die Verschwenkung ablesen, so dass auch hier eine genaue Dokumentation möglich ist.

Wie bereits erwähnt, ist die erste Aufnahme 10 oberhalb einer zweiten, auf der Grundplatte 2 geführten Aufnahme 12 für die Anschlussvorrichtung 13 angeordnet. Auf der Platte 2 ist die zweite Aufnahme 12 in einer horizontalen Ebene durch eine Einstellvorrichtung 19 verschiebbar, wobei hier der Übersichtlichkeit halber nur die Stangenführung in der der Zeichenebene entsprechenden Richtung dargestellt ist. Die zweite Aufnahme 12 ist folglich an einer verstellbaren Stange 20, welche arretierbar sein kann, angeordnet, welche wiederum in einem Schiebekörper 21 geführt ist. Der Schiebekörper 21 wiederum ist über eine hier nur angedeutete Stange 22 in der zur Zeichenebene senkrechten Ebene führbar.

Auf der Grundplatte 2 ist eine Skala aufgebracht, von der die momentane Position der zweiten Aufnahme 12 abgelesen werden kann und die eine Dokumentation ermöglicht.

Die erste Aufnahme 10 und die zweite Aufnahme 12 sind mithin über verschiedenste Mittel zur relativen Verschiebung und Verschwenkung aneinander gekoppelt, nämlich die Einstelleinrichtung 19, das Stativ 3, und die Gelenkverbindung 8. Die aktuelle Einstellung lässt sich zum Zwecke der Dokumentation über Messvorrichtungen, nämlich die Skala 15, die Winkelmesser 16 und die hier nicht sichtbare Skala auf der Grundplatte 2, zum Zwecke der Dokumentation feststellen bzw. ablesen.

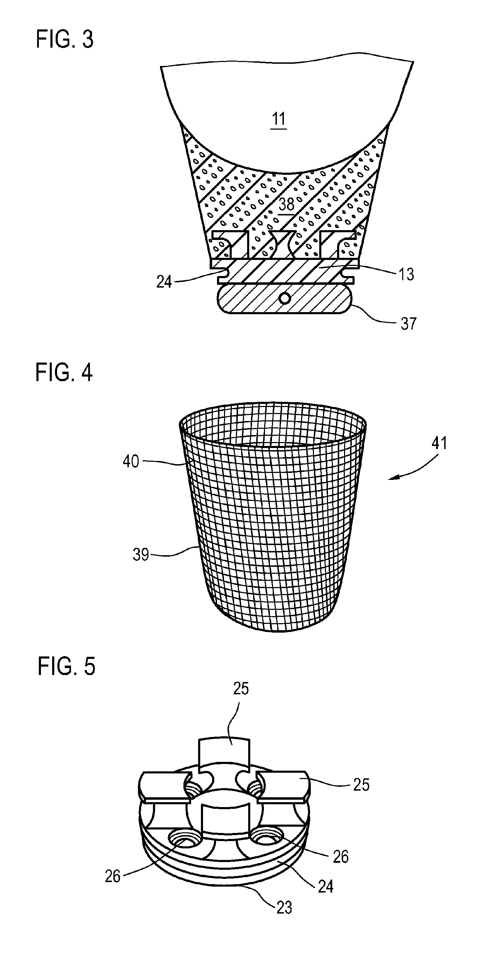

An dieser Stelle sei eine Beschreibung der verwendeten Anschlussvorrichtung 13 eingeschoben, vgl. hierzu auch die Fig. 5. Diese wird im Wesentlichen von einer runden Kunststoffplatte 23 gebildet, welche eine auf ihrer äußeren Oberfläche umlaufende Nut 24 umfasst. Ferner sind auf der dem Prothesenschaft 11 zuzuwendenden Seite mehrere symmetrische Stege 25 angeformt, die hinterschnitten sind. Diese dienen zur besseren, formschlüssigen Verbindung mit einem Füllmittel, was im Folgenden noch näher diskutiert werden wird. Schließlich weist die Anschlussvorrichtung 13 nur in Fig. 5 sichtbare Gewindelöcher 26 auf, die später der Befestigung eines Prothesenadapters und/oder einer Prothese dienen. Im vorliegenden Fall sind die Gewindelöcher 26 teils durch Stopfen verschlossen, teils dienen sie zur Fixierung der Anschlussvorrichtung 13 an der zweiten Aufnahme 12, wofür entsprechende Schraub- oder Klemmaufnahmen an der Oberseite der Aufnahme 12 vorgesehen sein können. Es sei an dieser Stelle angemerkt, dass die Anschlussvorrichtung 13 auch auf andere Weise an der zweiten Aufnahme 12 befestigt werden kann, beispielsweise mittels eines lösbaren Klebers oder einer anderen Klemmvorrichtung, insbesondere ist auch denkbar, lediglich eine Befestigung durch Haftreibung vorzusehen.

Die Befestigungsvorrichtung 1 umfasst ferner eine aus einem flexiblen Material bestehende Verbindungshülse 27, welche eine konisch zulaufende Form und einen an ihrem unteren Ende vorgesehenen Steg 28 aufweist, der der Nut 24 entspricht und zur zeitweisen Befestigung der Verbindungshülse 27 an der Anschlussvorrichtung 13 dient. Einmal befestigt, dichtet die mit ihren oberen Enden 29 an dem Prothesenschaft 11 anliegende Verbindungshülse 27 folglich einen Freiraum 30 zwischen dem Prothesenschaft 11 und der Anschlussvorrichtung 13 ab, so dass dieser mit einem Füllmittel, welches dann erhärtet, aufgefüllt werden kann. Daher weist die Verbindungshülse 27 auch die Form auf, die das Füllmittel, welches einen Teil der entstehenden festen Verbindung bildet, später annehmen soll.

Die Verbindungshülse 27 kann beispielsweise auf einer Seite aufklappbar sein, um sie entsprechend überzustülpen, wobei Schließmittel vorgesehen sein können, um sie auch dort abzudichten. Durch die Flexibilität der Verbindungshülse 27 ist es möglich, auch bei bereits angesetzter Verbindungshülse 27 noch eine relative Verschiebung und/oder Verschwenkung der in den Aufnahmen fixierten Teile, also des Prothesenschafts 11 und der Anschlussvorrichtung 13, zu erlauben.

Die Höhe der Verbindungshülse 27 ist dabei so zu wählen, dass sie für verschiedene über das Stativ 3 eingestellte Höhen geeignet ist.

Um das Füllmittel in den Freiraum 30 einbringen zu können, umfasst die Vorrichtung 1 ferner eine Spritzvorrichtung 31 mit daran angeschlossenem Schlauch 32, der in einem Stutzen 33 endet. Der Stutzen 33 kann in eine Einlassöffnung 34 der Verbindungshülse 27 eingeschoben werden, wozu im Detail auf die Fig. 2 verwiesen wird.

Dort lässt sich erkennen, dass der Freiraum 30 oben vom Prothesenschaft 11, unten von der Anschlussvorrichtung 13 und an den Seiten von der Verbindungshülse 27 begrenzt wird. Durch die Einlassöffnung 34 ist der Stutzen 33 des Schlauchs 32 eingesteckt, wobei die Einlassöffnung 34 im vorliegenden Falle mit einer nicht näher dargestellten Schließvorrichtung versehen ist, die beim Einstecken des Stutzens 33 geöffnet wird und beim Herausziehen des Stutzens 33 die Einlassöffnung 34 wieder verschließt.

Zum Auslassen der Luft beim Einspritzen des Füllmittels weist die Verbindungshülse 27 ferner Luftauslassöffnungen 35 auf, deren Größe so gewählt wurde, dass Luft aus dem Freiraum 30 entweichen kann, während dieser gefüllt wird, jedoch das Füllmittel sie nicht durchdringen kann, sondern die Luftauslassöffnungen 35 einfach verschließt, wenn die entsprechende Füllstandshöhe erreicht ist. Die Luftauslassöffnungen 35 sind auf verschiedenen Höhen zirkular umlaufend vorgesehen, mithin im Wesentlichen gleichmäßig verteilt.

Es sei an dieser Stelle angemerkt, dass selbstverständlich auch andere Lösungen zum Einspritzen des Füllmittels und zum Auslassen der Luft vorgesehen sein können. So können beispielsweise die Auslassöffnungen 35 zunächst auf einen innerhalb der Wandung der Verbindungshülse 27 verlaufenden Kanal münden, welcher anderenorts eine Öffnung zur Umgebung aufweist. Denkbar ist es auch, sowohl für das Einspritzen als auch für die Ableitung der entweichenden Luft, Schläuche von oben zwischen Prothesenschaft 11 und Verbindungshülse 27 einzuführen.

Mit der beschriebenen Vorrichtung 1 kann nun das erfindungsgemäße Verfahren vorteilhaft durchgeführt werden. Dafür werden zunächst, wie auch in den Figuren schon gezeigt, der Prothesenschaft 11 in der ersten Aufnahme 10 und die Anschlussvorrichtung 13 in der zweiten Aufnahme 12 fixiert. Sodann wird die Verbindungshülse 27 an der Anschlussvorrichtung 13 befestigt, wobei darauf hingewiesen sei, dass es auch möglich ist, insbesondere bei einer rundum geschlossenen Verbindungshülse 27, diese zuvor auf die Anschlussvorrichtung 13 aufzustülpen, also bevor der Prothesenschaft 11 von oben in sie eingesteckt wird und fixiert wird. Diese Fixierung kann in einer Grundstellung der Aufnahmen beispielsweise so erfolgen, dass die Längsrichtung der Prothesenschafts 11 exakt der Vertikalen entspricht und die Mittellinie des Prothesenschafts 11 den Mittelpunkt der runden Anschlussvorrichtung 13 schneidet.

Danach kann mit Hilfe der Mittel zur relativen Verschiebung und Verschwenkung, also mittels des Stativs 3, der Gelenkverbindung 8 und der Einstelleinrichtung 19 die relative Position und Ausrichtung des Prothesenschafts 11 und der Anschlussvorrichtung 13 so lange verändert werden, bis sie für den Patienten ideal ist und eine endgültige Position und Ausrichtung ausgewählt wurde. Die Messvorrichtungen, also die Skala 15, die Winkelmesser 16 und die auf der Grundplatte 2 vorgesehene Skala, werden benutzt, um die endgültige Position und Ausrichtung beschreibende Geometriedaten zu messen bzw. abzulesen, so dass eine Dokumentation erfolgen kann.

Während der Prothesenschaft 11 in der ersten Aufnahme 10 und die Anschlussvorrichtung 13 in der zweiten Aufnahme 12 in der endgültigen relativen Position und Ausrichtung gehalten werden, wird nun die feste Verbindung angefertigt. Als Füllmittel wird im vorliegenden Beispiel ein zweikomponentiger PU-Schaum verwendet, der mittels einer Zweikomponentenkartusche im Mischerrohr angemischt wird und mittels der Spritzvorrichtung 31 und dem Schlauch 32, dessen Stutzen 33 in die Einlassöffnung 34 eingesteckt ist, in den Freiraum 30, der von dem Prothesenschaft 11, der Anschlussvorrichtung 13 und der Verbindungshülse 27 rundum begrenzt wird, eingespritzt. Es kann auch vorgesehen sein, dass der PU-Schaum in einem separaten Behälter gemischt und anschließend in die Spritzvorrichtung 31 gefüllt wird oder er durch Schütteln der Spritzvorrichtung 31 vermischt wird. Zudem kann die Spritzvorrichtung 31 auch eine bei 36 optional angedeutete Mischeinrichtung enthalten.

Zur Verbesserung der Haftung des Füllmittels, also des zweikomponentigen PU-Schaums, mit dem Material des Prothesenschafts 11 und dem Material der Anschlussvorrichtung 13 wird dem PU-Schaum ein Lösemittel beigemengt, welches die Materialien des Prothesenschafts 11 und der Anschlussvorrichtung 13 leicht anlöst und damit die Haftfläche vergrößert. Die Verbindungshülse 27 bleibt jedoch unberührt. Es sei angemerkt, dass alternativ zur Zusetzung des Lösemittels auch die entsprechende Oberfläche des Prothesenschafts 11 und der Anschlussvorrichtung 13 angeraut werden kann.

Beim Einspritzen verdrängt das Füllmittel die Luft im Freiraum 30, wobei diese durch die kleinen Luftauslassöffnungen 35 der Verbindungshülse 27 entweicht. Hat das Füllmittel im Wesentlichen den oberen Rand des Freiraums 30 erreicht, kann die Spritzvorrichtung 31 von der Verbindungshülse 27 abgenommen werden. Die Einlassöffnung verschließt sich automatisch. Wenn eine derartige Funktionalität nicht vorgesehen ist, muss der Schlauch abgeklemmt werden.

Nachdem das Füllmittel ausgehärtet ist, kann die Verbindungshülse 27 abgenommen werden, da sich diese nicht mit dem Polyurethan-Schaum verbindet. Die Verbindungshülse 27 kann dann in einem weiteren Vorgang wiederverwendet werden, es ist jedoch auch möglich, für jeden Vorgang eine Verbindungshülse 27 vorzusehen.

Danach kann der Prothesenadapter 37 an die Anschlussvorrichtung 13 montiert werden, wie dies in Fig. 3 dargestellt ist. Zur Befestigung des Prothesenadapters 37 werden dabei die Gewindelöcher 26 genutzt. Das ausgehärtete Füllmittel 38 liegt zu diesem Zeitpunkt noch offen.

Auch die übrigen Prothesenbestandteile können nun aufgrund des modularen Prothesenadapters 37 angeschlossen werden und die Prothesenstellung kann beim Gehen und Stehen des Amputierten überprüft werden.

Schließlich ist es noch möglich, das gehärtete Füllmittel 38, also den PU-Schaum, vor Licht, Beschädigung und äußeren Witterungsumständen zu schützen. Hierzu wird eine Umhüllung verwendet, wie sie durch die Prinzipskizze in Fig. 4 dargestellt wird.

Die Schutzumhüllung 41 ist dabei als ein konisch zulaufender Schlauch bzw. Konus 39 aus einem flexiblen Gewebe 40 ausgebildet. Die Fäden des Gewebes 40 können dabei transparent sein. Die harzgetränkte Schutzumhüllung 41 wird in einem aufgerollten Zustand unterhalb der Anschlussvorrichtung 13 angesetzt und nach oben abgerollt. Dabei umschließt sie die Anschlussvorrichtung 13, das gehärtete Füllmittel 38 sowie einen Teil des Prothesenschafts 11. Die Nut 24 der Anschlussvorrichtung 13 wird nun genutzt, um die Umhüllung 41 darin mit einem harzgetränktem Faden oder einem anderen zugfesten Faden abzubinden. Dies dient neben dem Schutz des PU-Schaums auch zur Verbesserung der Haftung zwischen Prothesenschaft 11 und Anschlussvorrichtung 13, sowie zur Erhöhung der Festigkeit der so entstandenen Verbindung, die im hiesigen Beispiel dann von dem gehärteten Füllmittel 38 und der Umhüllung 41 gebildet ist.

## Patentansprüche

1. Vorrichtung (1) zur Befestigung einer Anschlussvorrichtung (13) für eine Prothese und/oder einen Prothesenadapter (37) an einem Prothesenschaft (11), **gekennzeichnet durch**:
- eine erste Aufnahme (10) für den Prothesenschaft (11),
- eine zweite, insbesondere unterhalb der ersten Aufnahme (10) angeordnete Aufnahme (12) für die Anschlussvorrichtung (13),
- Mittel zur relativen Verschiebung und/oder Verschwenkung des in der ersten Aufnahme (10) fixierten Prothesenschafts (11) und der in der zweiten Aufnahme (12) fixierten Anschlussvorrichtung (13) und
- Mittel zur festen Verbindung des fixierten Prothesenschafts (11) und der fixierten Anschlussvorrichtung (13) in einer gewünschten relativen Position und/oder Ausrichtung.

2. Vorrichtung nach Anspruch 1, **dadurch gekennzeichnet, dass** die Mittel zur festen Verbindung eine insbesondere an der Anschlussvorrichtung (13), insbesondere mittels eines einer Nut (24) der Anschlussvorrichtung (13) entsprechenden Steges (28), befestigbare, den Freiraum (30) zwischen dem in der ersten Aufnahme (10) fixierten Prothesenschaft (11) und der in der zweiten Aufnahme (12) fixierten Anschlussvorrichtung (13) abdichtende, flexible Verbindungshülse (27) umfassen.

3. Vorrichtung nach Anspruch 2, **dadurch gekennzeichnet, dass** die Verbindungshülse (27) eine der gewünschten endgültigen Form der Verbindung entsprechende Formgebung aufweist, insbesondere eine Kegelstumpfform und/oder eine Zylinderform, und/oder aus einem im Wesentlichen keine Haftverbindung mit einem Füllmittel (38) eingehenden Material besteht.

4. Vorrichtung nach einem der vorangehenden Ansprüche, **dadurch gekennzeichnet, dass** die Mittel zur relativen Verschwenkung und/oder Verschiebung eine Höheneinstelleinrichtung für die erste Aufnahme (10) und/oder wenigstens eine Schwenkvorrichtung für die erste Aufnahme (10) umfassen.

5. Vorrichtung nach einem der vorangehenden Ansprüche, **dadurch gekennzeichnet, dass** wenigstens eine Messvorrichtung zur Anzeige einer eingestellten relativen Positionierung und/oder Ausrichtung vorgesehen ist.

6. Verfahren zur Befestigung einer Anschlussvorrichtung für eine Prothese und/oder einen Prothesenadapter an einem Prothesenschaft, insbesondere unter Verwendung einer Vorrichtung gemäß einem der Ansprüche 1 bis 5, **dadurch gekennzeichnet, dass**
- die Anschlussvorrichtung und der Prothesenschaft in einer definierten relativen Position und/oder Ausrichtung zueinander jeweils in einer zweiten und einer ersten Aufnahme fixiert werden,
- durch relative Verschiebung und/oder Verschwenkung der Aufnahmen zueinander eine endgültige relative Position und/oder Ausrichtung eingestellt wird, und
- die fixierte Anschlussvorrichtung und der fixierte Prothesenschaft unter Bildung einer festen Verbindung aneinander in der endgültigen relativen Position und/oder Ausrichtung befestigt werden.

7. Verfahren nach Anspruch 6, **dadurch gekennzeichnet, dass** die erste, oberhalb der zweiten Aufnahme liegende Aufnahme verschwenkt und/oder höhenverstellt wird und/oder die zweite Aufnahme in einer horizontalen Ebene verschoben wird.

8. Verfahren nach einem der Ansprüche 6 oder 7, **dadurch gekennzeichnet, dass** mittels wenigstens einer Messvorrichtung die endgültige relative Position und/oder Ausrichtung beschreibende Geometriedaten gemessen und/oder abgelesen werden.

9. Verfahren nach einem der Ansprüche 6 bis 8, **dadurch gekennzeichnet, dass** ein zwischen der fixierten Anschlussvorrichtung und dem fixierten Prothesenschaft liegender Freiraum mit einem Füllmittel, insbesondere einem PU-Schaum. aufgefüllt wird, welches in ausgehärtetem Zustand wenigstens einen Teil der Verbindung bildet.

10. Verfahren nach Anspruch 9, **dadurch gekennzeichnet, dass** als Form für das Füllmittel eine insbesondere zylindrische und/oder kegelstumpfförmige, elastische Verbindungshülse zur Abdichtung des Freiraums verwendet und insbesondere an der Anschlussvorrichtung, insbesondere mittels eines einer an der Anschlussvorrichtung ausgebildeten Nut entsprechenden Steges, befestigt wird.

11. Verfahren nach Anspruch 9 oder 10, **dadurch gekennzeichnet, dass** zum Schutz des Füllmittels dieses mit einer Schutzumhüllung versehen wird, insbesondere ein bevorzugt harzgetränkter Schlauch oder Konus aus einem flexiblen Gewebe wenigstens auf das erhärtete Füllmittel, insbesondere auch auf die Aufnahme und/oder wenigstens einen Teil des Prothesenschafts, aufgerollt wird.

12. Verfahren nach einem der Ansprüche 6 bis 11, **dadurch gekennzeichnet, dass** ein insbesondere modularer Prothesenadapter an die Anschlussvorrichtung montiert wird.

13. Schutzumhüllung (41) für eine ein ausgehärtetes Füllmaterial (38) umfassende Verbindung eines Prothesenschafts (11) mit einer Anschlussvorrichtung (13) für eine Prothese und/oder einen Prothesenadapter (37), wobei die Verbindung insbesondere mit der Vorrichtung (1) nach einem der Ansprüche 1 bis 5 und/oder nach dem Verfahren gemäß einem der Ansprüche 6 bis 12 hergestellt wurde, **dadurch gekennzeichnet, dass** die aus einem elastischen Gewebe (40) bestehende, insbesondere schlauch-oder konusförmige Schutzumhüllung (41) auf die Verbindung aufziehbar, insbesondere aufrollbar, ist.

14. Schutzumhüllung nach Anspruch 13, **dadurch gekennzeichnet, dass** sie auch zur Überdeckung wenigstens eines Teils der Anschlussvorrichtung (13) und/oder wenigstens eines Teils des Prothesenschafts (11) dimensioniert ist.
